# EUROPÄISCHE PATENTSCHRIFT

(11) **EP 2 911 601 B1**
(45) Veröffentlichungstag und Bekanntmachung des Hinweises auf die Patenterteilung: **07.12.2016**
(21) Anmeldenummer: 13783535.1
(22) Anmeldetag: 25.10.2013
(51) Int. Cl.: A61B 17/70, A61B 17/86, A61B 90/00

(54) **KNOCHENSCHRAUBE**
BONE SCREW
VIS À OS

(30) Priorität: 26.10.2012 DE 102012219630
(43) Veröffentlichungstag der Anmeldung: 02.09.2015
(73) Patentinhaber: Premiere Medical GmbH, 89155 Erbach (DE)
(72) Erfinder: VAZIFEHDAN, Farsam, 70195 Stuttgart (DE); WILLMANN, Nicolas, 89077 Ulm (DE); SCHÖNHÖFFER, Helmut, 89155 Erbach (DE)
(74) Vertreter: Kohler Schmid Möbus Patentanwälte
(86) Internationale Anmeldenummer: PCT/EP2013/072398
(87) Internationale Veröffentlichungsnummer: WO 2014/064253

(56) Entgegenhaltungen:
- US-A- 5 733 286
- US-A1- 2002 013 585
- US-A1- 2007 093 827
- US-A1- 2011 208 248

## Beschreibung

Die Erfindung betrifft eine Knochenschraube für Implantate zur Korrektur und Stabilisierung der Wirbelsäule.

Bei der operativen Instrumentierung der Wirbelsäule werden in der klinischen Praxis Knochenschrauben mit einem am Schraubenkopf befestigten Kopfgehäuse eingesetzt. Das Kopfgehäuse weist eine Stabaufnahme für einen Fixierstab auf, der einem Aufrichten und/oder gegenseitigen Stabilisieren bzw. Führen von Wirbelkörpern dient.

Die Kopfgehäuse sind üblicher Weise als sogenannte Tulpenköpfe ausgebildet. Bei den sogenannten monoaxialen Knochenschrauben sind die Kopfgehäuse am Schraubenkopf drehfest oder einachsig verdrehbar angeordnet. Bei den sogenannten polyaxialen Knochenschrauben können die Kopfgehäuse gegenüber der Längsachse des Gewindeschafts ein- oder mehrachsig ausgelenkt werden.

US 2007/093827A1 zeigt eine polyaxiale Knochenschraube für Implantate zur Korrektur und Stabilisierung der Wirbelsäule umfassend einen Gewindeschaft mit einem Schraubenkopf, der ein Antriebsprofil für ein Drehwerkzeug aufweist. Ein Kopfgehäuse der Knochenschraube weist eine Stabaufnahme für einen Fixierstab auf, wobei das Kopfgehäuse am Schraubenkopf relativ zur Längsachse der Knochenschraube auslenkbar und um seine Gehäuselängsachse frei drehbar am Schraubenkopf angeordnet ist. Das Kopfgehäuse ist über einen geschlitzten Federring am Schraubenkopf axial lagegesichert, wobei das Kopfgehäuse im Bereich seiner Schraubenkopfaufnahme innenseitig eine Ringnut aufweist, in die der Federring beim axialen Einführen des Schraubenkopfs in die Schraubenkopfaufnahme des Kopfgehäuses hinein ausweichen kann. Innerhalb der Ringnut ist in deren gewindeschaftseitigen Bereich ein Vorsprung für eine radiale Begrenzung einer Aufweitung des Federrings angeordnet. Die Schraubenkopfaufnahme ist mit einem axialen Anschlag für den Federring versehen, der durch einen Seitenwandvorsprung des Kopfgehäuses gebildet ist und durch den der Federring gegenüber einem axialen Entfernen oder Herausfallen aus der Schraubenkopfaufnahme des Kopfgehäuses gesichert ist.

Weitere Knochenschrauben sind aus der US 2002/013585 A1 sowie aus der US 5 733 286 A bekannt.

In der Praxis gestaltet sich das Auffinden des Antriebsprofils der Knochenschraube mit einem zum Ein- oder Ausdrehen eingesetzten Drehwerkzeug nicht zuletzt aufgrund der etablierten minimal- oder mikroinvasiven Operationstechniken schwierig. Dies ist insbesondere bei einer intraoperativen Korrektur der intraossären Lage der Knochenschraube der Fall und kann zu einer unerwünschten Traumatisierung der umgebenden Weichteile führen. Knochenschrauben müssen in ihrem implantierten Zustand darüber hinaus hohen mechanischen Belastungen standhalten. Insbesondere muss die Befestigung des Kopfgehäuse am Schraubenkopf auf die hohen mechanischen Belastungen ausgelegt sein. Die am Markt verfügbaren Knochenschrauben weisen deshalb oftmals einen komplexen konstruktiven Aufbau sowie einen hohen Montageaufwand auf. Dies ist unter Kostenaspekten nachteilig.

US 2011/208248 A1 zeigt eine Knochenschraube mit einem Schraubenkopf, der ein Antriebsprofil für ein Drehwerkzeug aufweist. Das Antriebsprofil ist von einem Einsatzteil gebildet, ist, das in einer Lageraufnahme des Schraubenkopfs angeordnet ist, wobei das Einsatzteil am Kopfgehäuse geführt gehalten und gemeinsam mit diesem gegenüber der Längsachse des Gewindeschafts der Knochenschraube auslenkbar ist. Das Antriebsprofil der Knochenschraube kann dadurch mit einem zum Ein- oder Ausdrehen der Knochenschraube eingesetzten Drehwerkzeug vereinfacht aufgefunden werden, wodurch einer intraoperative Gewebetraumatisierung entgegengewirkt werden kann.

Aufgabe der Erfindung ist es, eine Knochenschraube anzugeben, die bei vereinfachter Handhabung eine nochmals geringere Gewebetraumatisierung selbst bei minimalinvasiven operativen Zugängen ermöglicht. Die Knochenschraube soll eine hohe mechanische Belastbarkeit aufweisen und zugleich kostengünstig herzustellen sein und einen geringen Montageaufwand aufweisen.

Die die polyaxiale Knochenschraube betreffende Aufgabe wird durch eine Knochenschraube mit den in Patentanspruch 1 angegebenen Merkmalen gelöst.

Bei der erfindungsgemäßen Knochenschraube ist das Antriebsprofil der Knochenschraube unabhängig von einer Auslenkung (einem Verkippen/Abklappen) des Kopfgehäuses gegenüber der Längsachse des Gewindeschafts der Knochenschraube zu jedem Zeitpunkt mit der Gehäuseachse des Kopfgehäuses ausgerichtet bzw. fluchtet mit dieser. Ein Drehwerkzeug kann dadurch einfacher, zügiger und sicherer an das Antriebsprofil der Knochenschraube herangeführt und mit diesem drehschlüssig verkoppelt werden. Ein zeitraubendes, traumatisierendes und die Hardware ggf. beschädigendes Aufsuchen eines zum Ankoppeln an das Antriebsprofil geeigneten Ansetzwinkels des Drehwerkzeugs am Schraubenkopf der Knochenschraube entfällt. Einer übermäßigen Traumatisierung des umgebenden Weichteilgewebes kann dadurch selbst bei einem minimalinvasiven operativen Zugangsweg zuverlässig entgegengewirkt werden. Dadurch, dass das Kopfgehäuse über einen Federring mit dem Schraubenkopf der Knochenschraube unlösbar verrastet ist, kann das Kopfgehäuses darüber hinaus an dem Schraubenkopf besonders einfach montiert werden. Der Federring ist geschlitzt ausgeführt und ist in radialer Richtung innenspannend ausgeführt. Durch den Federring wird eine freie Verdrehbarkeit des Kopfgehäuses um seine Gehäuselängsachse relativ zum Schraubenkopf gewährleistet. Das Kopfgehäuse weist eine Schraubenkopfaufnahme auf, deren Seitenwand mit einer Ringnut versehen ist. Die Ringnut ermöglicht, dass der Federring bei einem axialen Aufstecken des Kopfgehäuses auf den Schraubenkopf in radialer Richtung ausweichen kann, um eine Passage (insbesondere der Äquatorialebene) des Schraubenkopfs durch den Federring hindurch zu ermöglichen. Innerhalb der Ringnut ist ein Vorsprung für eine radiale Begrenzung einer Aufweitung des Federrings angeordnet. Der Vorsprung ist dabei vorzugsweise in einem unteren (gewindeschaftseitigen) Bereich der Nut angeordnet. Der Vorsprung kann insbesondere ringförmig umlaufend ausgebildet sein. Die unlösbare Rastverbindung zwischen dem Kopfgehäuse und dem Schraubenkopf kann nicht zerstörungsfrei gelöst werden. Ein unerwünschtes axiales Entfernen bzw. Herausfallen des Federrings aus der Schraubenkopfaufnahme des Kopfgehäuses wird erfindungsgemäß dadurch unterbunden, dass die Schraubenkopfaufnahme mit einem axialen Anschlag für den Federring versehen ist. Der Anschlag ist durch einen Seitenwandvorsprung, beispielsweise einen Ringbund, eine Nutflanke oder dergl., gebildet. Die Knochenschraube kann als Wirbelkörperschraube zum Einschrauben in den Wirbelkörper eines Wirbels bzw. als sogenannte Pedikelschraube ausgeführt sein.

Das Einsatzteil weist zwei (erste) Lagerzapfen auf, die sich jeweils in radialer Richtung vom Einsatzteil wegerstrecken.

Das Einsatzteil ist an einem in der Lageraufnahme verschwenkbar angeordneten Kardanring gelagert. Die ersten Lagerzapfen des Einsatzteils greifen dabei in jeweils eine Seitenwandausnehmung des Kardanrings ein. Dadurch kann insgesamt ein nochmals vergrößerter Bewegungsumfang des Einsatzteils bzw. des Kopfgehäuses relativ zur Längsachse des Gewindeschafts der Knochenschraube realisiert werden. Darüber hinaus ergibt sich dadurch eine besonders leichtgängige Lagerung des Einsatzteils am Schraubenkopf. Dies ist für ein in der klinischen Praxis oftmals wesentliches feinfühliges Ausrichten des Kopfgehäuses von Vorteil. Der Kardanring greift vorzugsweise mit zweiten Lagerzapfen in Seitenwandausnehmungen des Schraubenkopfs ein, die zum (kopfseitigen) freien Ende der Knochenschraube hin vorzugsweise offen sind.

Das Einsatzteil kann nach der Erfindung zumindest abschnittsweise eine sphärische Außenkontur bzw. Außenseite und die Lageraufnahme des Schraubenkopfs zumindest abschnittsweise eine, insbesondere dazu korrespondierend ausgebildete, gewölbte Seitenwand aufweisen. Dies ist für die mechanische Belastbarkeit der Einsatzteil-Schraubenkopfkopplung von Vorteil. Darüber hinaus kann die Lageraufnahme des Schraubenkopfs beim Festsetzen eines Fixierstabs in der Stabaufnahme durch das Einsatzteil in radialer Richtung aufgeweitet und dadurch mit dem Kopfgehäuses verklemmt werden. Im Hinblick auf eine optimierte Klemmwirkung hat es sich als vorteilhaft erwiesen, wenn die Außenkontur bzw. Außenseite des Einsatzteils vielfach facettiert ausgeführt ist. Der Schraubenkopf kann nach der Erfindung darüber hinaus mehrfach geschlitzt sein, um ein gleichmäßiges Aufdehnen (Aufweiten) des Schraubenkopfes zu ermöglichen.

Das Einsatzteil kann erfindungsgemäß insbesondere mit einem ringbundförmigen Führungsabschnitt am Kopfgehäuse innenseitig anliegen. Dadurch kann das Kopfgehäuse überdies um seine Gehäuselangsachse gegenüber dem Schraubenkopf frei verdreht werden.

Unter fertigungstechnischen Gesichtspunkten ist das Einsatzteil vorteilhaft mittels des Kopfgehäuses in der Lageraufnahme des Schraubenkopfs axial lagegesichert. Auf zusätzliche Sicherungselemente kann dadurch verzichtet werden.

Bei einer langstreckigen offenen Instrumentierung der Wirbelsäule (="Mehretageninstrumentierung"), wie diese beispielsweise bei Operationen zur Korrektur von Wirbelsäulendeformitäten, insbesondere einer Skoliose, erforderlich ist, muss der eingesetzte Fixierstab in der Stabaufnahmen einer Vielzahl von Knochenschrauben positioniert und fixiert werden. Die in der Praxis eingesetzten Fixierstäbe sind in der Regel äußerst biegesteif und werden bevorzugt außerhalb des Körpers in eine gewünschte Form vorgebogen. Sobald der Fixierstab in der Stabaufnahme einer andere Knochenschraube gehalten angeordnet ist, kann dieser oftmals nur unter hohem Aufwand, beispielsweise mittels eines sogenannten "rod-benders", nachträglich in Form gebogen bzw. in die Stabaufnahme herkömmlicher Lang-Kopfgehäuse eingebracht werden. Dazu muss der Fixierstab in der Regel über einen der beiden Wandschenkel des Kopfgehäuses (hinweg-)gehebelt werden. Die erfindungsgemäße Weiterbildung der Knochenschraube gemäß Patentanspruch 7 erlaubt durch die unterschiedlich langen Wandschenkel der Stabaufnahme des Kopfgehäuses demgegenüber ein kraftschonendes, wenig traumatisches und vereinfachtes Einbringen und Fixieren des Fixierstabs in der Stabaufnahme mittels eines an die beiden Wandschenkel ankoppelbaren Repositionswerkzeugs. Erforderliche Operationszeiten können dadurch insgesamt verkürzt und zeitraubende sowie häufig traumatisierende Revisionen an der Instrumentierung vermieden werden. So kann der Fixierstab mit einem Repositionsinstrument vereinfacht in einer zur Gehäuselängsachse des Kopfgehäuses radialen Richtung über den kürzeren der beiden Wandschenkel in Richtung auf den längeren Wandschenkel des Kopfgehäuses der Knochenschraube gehebelt und mit dem Repositionsinstrument nachfolgend in der Stabaufnahme abgesenkt sowie mittels einer Fixierschraube an der Knochenschraube festgesetzt werden.

Der längere zweite Wandschenkel des Kopfgehäuses kann nach dem Festlegen bzw. Fixieren des Fixierstabs im Kopfgehäuse mit einfachen Mitteln gekürzt werden. Dies kann beispielsweise mit einem Schneid- oder einem Trennwerkzeug erfolgen.

Nach einer besonders bevorzugten Weiterbildung der Erfindung weist der längere Wandschenkel des Kopfgehäuses eine Sollbruchstelle auf. Dadurch kann der längere zweite Wandschenkel vereinfacht mittels einer Zange oder dergl. im Bereich der Sollbruchstelle gebrochen und so auf ein erforderliches Mindestmaß gekürzt werden.

Nach der Erfindung kann die Sollbruchstelle zumindest eine umfangsseitige Kerbe des zweiten Wandschenkel umfassen. Für ein besonders atraumatisches Kürzen des zweiten Wandschenkels hat es sich als vorteilhaft erwiesen, wenn der Wandschenkel innen- und außenseitig mit einer solchen Kerbe versehen ist. Die Sollbruchstelle kann auch durch eine anders geartete Materialschwächung des zweiten Wandschenkels erzeugt sein.

Das Innengewindesegment des ersten Wandschenkels kann sich bevorzugt in axialer Richtung bis zu dessen freien Rand und das Innengewindesegment des zweiten Wandschenkels bis zu dessen Sollbruchstelle erstrecken. Dadurch weist das Kopfgehäuse im implantierten Zustand eine besonders kompakte Bauhöhe auf.

Nachfolgend wird die Erfindung anhand eines in der Zeichnung wiedergegebenen Ausführungsbeispiels näher erläutert.

Die gezeigte und beschriebene Ausführungsform ist nicht als abschließende Aufzählung zu verstehen, sondern hat vielmehr beispielhaften Charakter für die Schilderung der Erfindung.

In der Zeichnung zeigen:
- Fig. 1: eine Knochenschraube mit einem am Schraubenkopf der Knochenschraube polyaxial angelenkten Kopfgehäuse, das eine Stabaufnahme für einen Fixierstab aufweist, wobei ein Antriebsprofil der Knochenschraube von einem Einsatzkörper gebildet ist, der in einer Aufnahme des Schraubenkopfs angeordnet und gemeinsam mit dem Kopfgehäuse gegenüber dem Gewindeschaft der Knochenschraube auslenkbar ist, in Seitenansicht;
- Fig. 2: die Knochenschraube aus Fig. 1, in einer anderen Seitenansicht;
- Fig. 3: die Knochenschraube aus Fig. 1, in einer perspektivischen Ansicht;
- Fig. 4: die Knochenschraube aus Fig. 1, in einer explodierten Darstellung;
- Fig. 5: die Knochenschraube aus Fig. 1, in einem Längsschnitt;
- Fig. 6: die Knochenschraube aus Fig. 1 bei der das Kopfgehäuse gegenüber der Längsachse des Gewindeschafts der Knochenschraube abgeklappt ist, im Längsschnitt;
- Fig. 7: eine Knochenschraube, bei der das Antriebsprofil der Knochenschraube von einem Einsatzteil gebildet ist, das am Schraubenkopf der Knochenschraube kardanisch gelagert ist;
- Fig. 8: die Knochenschraube aus Fig. 7 bei entferntem Kopfgehäuse, in einer perspektivischen Ansicht;
- Fig. 9: eine Knochenschraube mit einem Kopfgehäuse, deren Stabaufnahme von zwei Wandschenkeln mit unterschiedlicher Länge begrenzt ist, wobei einer der beiden Schenkel mit einer Sollbruchstelle versehen ist, in Seitenansicht;
- Fig. 10: die Knochenschraube aus Fig. 9, in einer anderen Seitenansicht;
- Fig. 11: die Knochenschraube aus Fig. 9 in einer perspektivischen Ansicht;

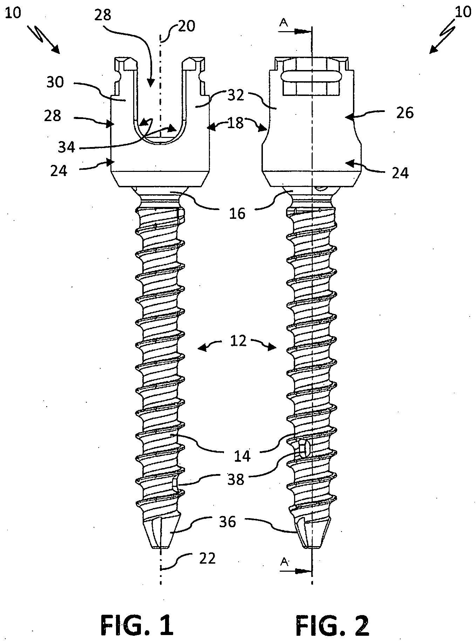

In den **Figuren 1** und **2** ist eine Knochenschraube **10** für Implantate zur Korrektur und Stabilisierung der Wirbelsäule in Seitenansicht gezeigt. Die Knochenschraube 10 umfasst einen Gewindeschaft **12** mit einem Gewinde **14,** einem Schraubenkopf **16** sowie ein am Schraubenkopf 16 polyaxial angelenktes Tulpen- oder Kopfgehäuse **18.** D. h., das Kopfgehäuse 18 kann mit seiner Gehäuselängsachse **20** gegenüber der Längsachse **22** des Gewindeschafts 12 der Knochenschraube 12 (mehrachsig) frei ausgelenkt und gegenüber dem Schraubenkopf 16 um die Längsachse 22 des Gewindeschafts 12 der Knochenschraube 10 frei verdreht werden.

Das Kopfgehäuse 18 weist einen ringförmigen Kopplungsabschnitt **24** und einen Aufnahmeabschnitt **26** auf. Der Kopplungsabschnitt 24 dient einem Verkoppeln des Kopfgehäuses 18 mit dem Schraubenkopf 16 der Knochenschraube 12. Der Aufnahmeabschnitt 26 weist eine Stabaufnahme **28** für einen nicht näher wiedergegebenen Fixierstab auf, wie dieser in der Praxis beispielsweise zur interpedikulären Instrumentierung der Wirbelsäule (Spondylodese) eingesetzt wird. Die Stabaufnahme 28 des Kopfgehäuses 18 ist durch einen ersten und einen zweiten Wandschenkel **30, 32** seitlich, d.h. in radialer Richtung zur Gehäuselängsachse 20, begrenzt. Die beiden Wandschenkel 30, 32 erstrecken sich parallel zur Gehäuselängsachse 20 vom Kopplungsabschnitt 24 weg und weisen Innengewindesegmente **34** auf, in die in bekannter Weise eine nicht näher wiedergegebene Fixierschraube zum Fixieren des Fixierstabs eingeschraubt werden kann. Die Innengewindesegmente 34 können selbsthemmend ausgebildet sein. Der Gewindeschaft 12 ist als Hohlschaft ausgebildet und weist vorliegend eine optionale Bohrspitze **36** auf. Der Gewindeschaft 12 der Knochenschraube 10 kann, wie in den Figuren 1 und 2 wiedergegeben, eine oder mehrere seitliche Ausführungsöffnungen **38** aufweisen, die das Einbringen von Fremdmaterial in ein den Gewindeschaft 12 umgebendes Knochengewebe erlauben.

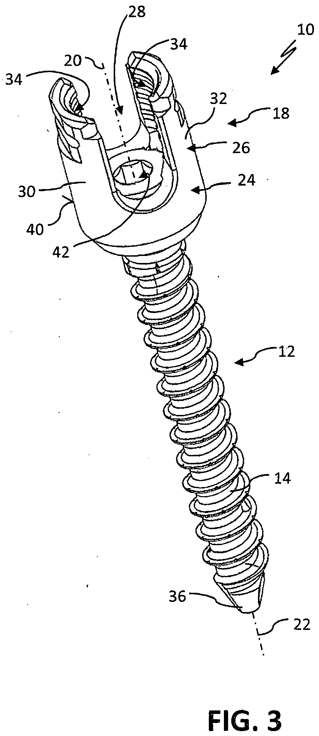

**Fig. 3** zeigt die Knochenschraube 10 in einer perspektivischen Ansicht. Das Kopfgehäuse 18 weist eine kreiszylindrische Außenmantelfläche **40** auf. Ein Antriebsprofil **42** der Knochenschraube 10 dient dem Angriff eines nicht näher wiedergegebenen Drehwerkzeugs, mit dessen Hilfe die Knochenschraube 10 in den Pedikel oder den Wirbelkörper eines Wirbels (nicht gezeigt) eingedreht bzw. aus diesem wieder herausgedreht werden kann.

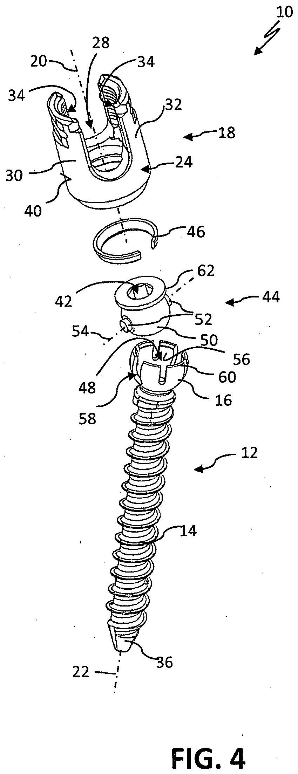

In **Fig. 4** ist die Knochenschraube 10 in einer explodierten Darstellung wiedergegeben. Die Knochenschraube 10 weist den Gewindeschaft 12, ein Einsatzteil **44** mit dem Antriebsprofil 42, das Kopfgehäuse 18 und einen geschlitzten Federring **46** auf.

Der Schraubenkopf 16 ist sphärisch ausgebildet und weist eine gewölbte Lageraufnahme **48** für das Einsatzteil 44 auf. Das Einsatzteil 44 weist einen zu der Lageraufnahme 48 im Wesentlichen korrespondierend ausgeformte kugelförmige Außenseite **50** auf. Von dem Einsatzteil 44 stehen zwei erste Lagerzapfen **52** seitlich weg, die auf einer gemeinsamen Querachse **54** angeordnet sind. Die Lageraufnahme 48 ist von einer Seitenwand **56** begrenzt, die Seitenwandausnehmungen **58** zur Aufnahme der Lagerzapfen 56 aufweist. Die Seitenwandausnehmungen 58 sind zum freien Rand **60** des Schraubenkopfs 16 hin offen.

Das Einsatzteil weist einen ringbundförmigen Führungsabschnitt **62** auf, über den das Einsatzteil 44 im montierten Zustand der Knochenschraube 10 am Kopfgehäuse 18 seitlich geführt ist. Das Antriebsprofil 42 des Einsatzteils 44 ist vorliegend als Innensechskant ausgeführt, kann aber auch andersartig profiliert sein.

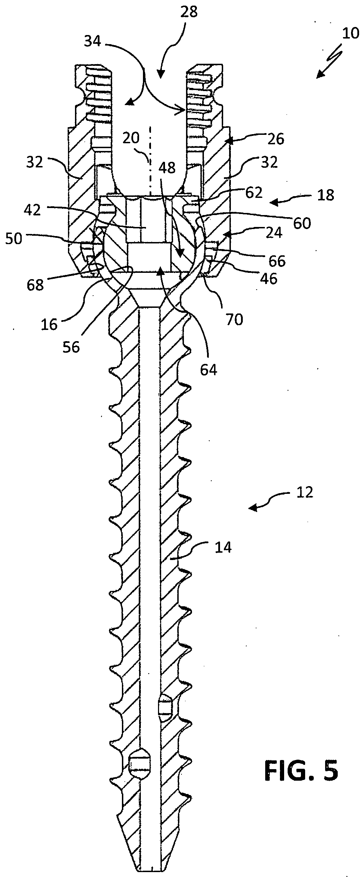

**Fig. 5** zeigt die Knochenschraube 10 im Längsschnitt entlang der in Fig. 2 mit A-A bezeichneten Schnittlinie. Das Kopfgehäuse 18 ist gegenüber dem Schraubenkopf 16 in seiner Neutralstellung angeordnet. In Neutralstellung fallen die Gehäuselängsachse 20 und die Längsachse 22 der Knochenschraube 10 zusammen, d.h., das Kopfgehäuse 18 ist gegenüber der Längsachse 22 nicht abgeklappt.

Das Einsatzteil 44 ist in der Lageraufnahme 48 des Schraubenkopfs 16 angeordnet und liegt an der Seitenwand 56 mit seiner sphärischen Außenseite 50 im Gleitspiel-Formschluss an. Die beiden ersten Lagerzapfen 52 sind in den Seitenwandausnehmungen des Schraubenkopfs (Fig. 4) angeordnet, so dass das Einsatzteil 44 mit dem Schraubenkopf 16 der Knochenschraube 10 bezüglich der Längsachse 22 der Knochenschraube 10 drehfest verkoppelt ist.

Das Einsatzteil 44 ragt in axialer Richtung aus der Lageraufnahme 48 hervor und erstreckt sich in das Kopfgehäuse 18 hinein. Das Einsatzteil 44 ist mit seinem ringbundförmigen Führungsabschnitt 62 in radialer Richtung an dem Kopfgehäuse 18 geführt gehalten.

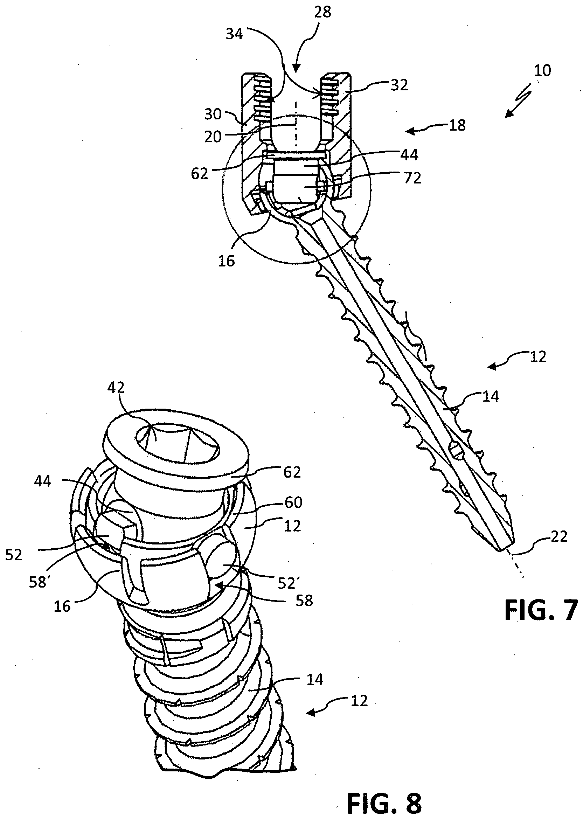

Das Kopfgehäuse 18 ist über den Federring 46 am Schraubenkopf 16 der Knochenschraube 10 axial lagegesichert. Das Kopfgehäuse 18 ist im Bereich ihrer Schraubenkopfaufnahme **64** innenseitig mit einer Ringnut **66** versehen, die konzentrisch zur Gehäuselängsachse 20 angeordnet ist. Die Ringnut kann auch, insbesondere bei einem abgewinkelt ausgeführten Kopfgehäuse18 (vgl. Fig. 7) gegenüber der Längsachse 22 verkippt angeordnet sein. Der Federring 46 kann bei einem axialen Einführen des Schraubenkopfs 16 in die Schraubenkopfaufnahme 64 des Kopplungsabschnitts 24 durch den Schraubenkopf geweitet und in radialer Richtung in die Ringnut 66 hinein ausweichen. Nach einem Passieren der Äquatorialebene des Schraubenkopfs 16 rückt der Federring 46 selbsttätig aus der Ringnut 66 aus. Ein innerhalb der Ringnut angeordneter Vorsprung **68** dient einer radialen Begrenzung einer (erneuten) Aufweitung des Federrings. Der Vorsprung 68 ist im unteren Bereich der Nut angeordnet. Der Federring fällt bei einer Montage der Knochenschraube und lotrechter Ausrichtung der Knochenschraube axial nach unten (=Fallring) und liegt dann an dem Vorsprung 68 an. Der Federring 46 ist durch einen Seitenwandvorsprung **70** des Kopfgehäuses 18 gegenüber einem axialen Entfernen aus der Schraubenkopfaufnahme 64 des Kopfgehäuses 18 gesichert.

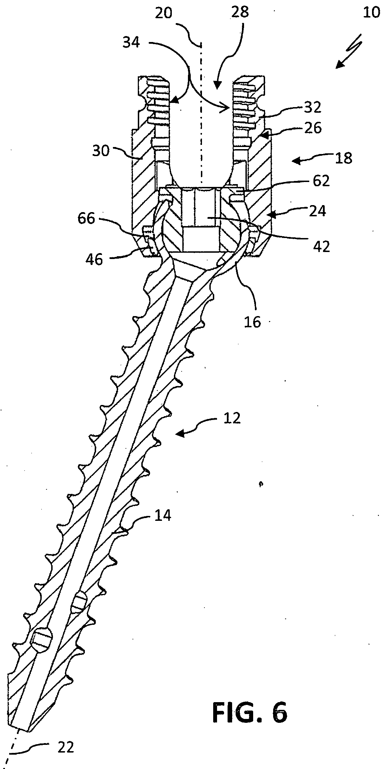

**Fig. 6** zeigt die Knochenschraube 10 mit einem gegenüber der Längsachse 22 der Knochenschraube 10 abgewinkelten (abgeklappten) Kopfgehäuse 18. Das Einsatzteil 44 ist mit seinem Antriebsprofil 42 entlang der Gehäuselängsachse 20 ausgerichtet und über die Stabaufnahme 28 mit einem Drehwerkzeug unmittelbar zugänglich.

**Fig. 7** zeigt eine Knochenschraube 10 im Längsschnitt, bei der das Einsatzteil 44 über einen (zusätzlichen) Kardanring **72** am Schraubenkopf 16 der Knochenschraube 12 verschwenkbar gelagert ist. Das Kopfgehäuse 18 entspricht im Wesentlichen dem Kopfgehäuse 18 der vorstehend erläuterten Knochenschraube.

In **Fig. 8** ist die Knochenschraube 10 in einem vergrößertem Teilausschnitt und in perspektivischer Ansicht gezeigt. Das Kopfgehäuse ist aus Darstellungsgründen nicht gezeigt. Das Einsatzteil 44 ist mit seinen beiden ersten Lagerzapfen 52 in nach oben offenen Seitenwandausnehmungen 58 des Kardanrings 72 gehalten angeordnet. Der Kardanring 72 weist seinerseits ein Paar zweite Lagerzapfen 52' auf, die sich in die Seitenwandausnehmungen der 58 des Schraubenkopfes 16 der Knochenschraube 10 hinein erstrecken. Der Aufbau der Knochenschraube 10 entspricht im Wesentlichen dem Aufbau der vorstehend beschriebenen Knochenschrauben.

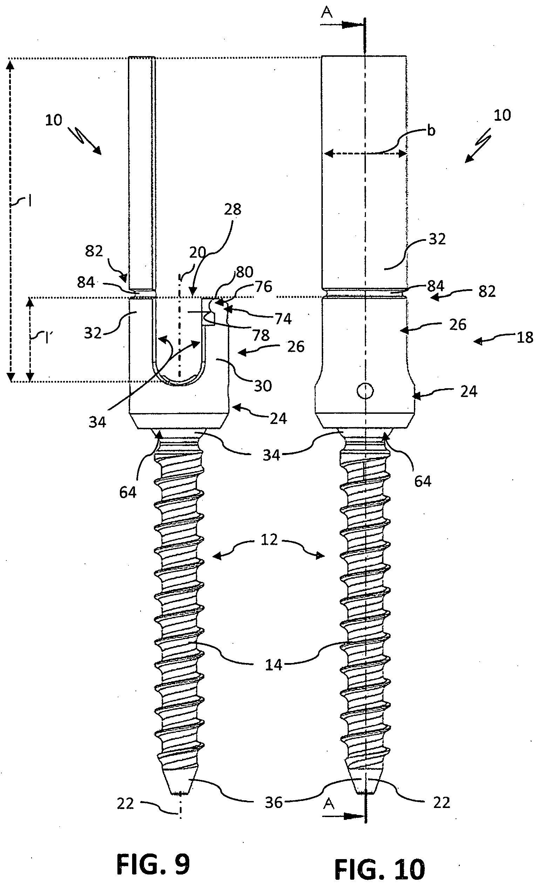

Die **Fign. 9** und **10** zeigen eine weitere Knochenschraube 10 in einer Seitenansicht. Die Knochenschraube 10 weist ein Kopfgehäuse 18 auf, das speziell auf den Gebrauch bei langstreckigen offenen Instrumentierung der Wirbelsäule, insbesondere zur Korrektur von Wirbelsäulendeformitäten, ausgerichtet und auch für minimalchirurgische Operationstechniken geeignet ist.

Das Kopfgehäuse 18 weist wie vorstehend beschrieben einen Kopplungsabschnitt 24 und einen Aufnahmeabschnitt 26 auf. Der Kopplungsabschnitt 26 weist eine Schraubenkopfaufnahme 64 für den zumindest abschnittsweise kugelförmigen Schraubenkopf 16 der Knochenschraube 10 auf. Das Kopfgehäuse 18 kann grundsätzlich auch einen andersartig ausgebildeten Kopplungsabschnitt zum Verkoppeln des Kopfgehäuses mit einer Knochenschraube aufweisen.

Die Stabaufnahme 28 ist durch einen ersten und einen zweiten Wandschenkel 30, 32 seitlich begrenzt, die voneinander beabstandet angeordnet sind. Die beiden Wandschenkel 30, 32 erstrecken sich parallel zur Gehäuselängsachse 20 vom Kopplungsabschnitt 24 weg. Die beiden Wandschenkel 30, 32 weisen Innengewindesegmente 34 auf, in die eine nicht näher wiedergegebene Fixierschraube zum Fixieren des Fixierstabs eingeschraubt werden kann.

Der erste Wandschenkel 30 weist an seinem freien Randabschnitt **74** zwei Kopplungsmittel **76** zum Ankoppeln eines weiter unten erläuterten Repositionsinstruments auf. Die Kopplungsmittel 76 sind bei dem gezeigten Ausführungsbeispiel als zungenartige Wandfortsätze ausgebildet, die in Umfangsrichtung zu jeweils einer Seite des Innengewindesegments 34 des ersten Wandschenkels 30 angeordnet sind. Die beiden Kopplungsmittel 76 weisen jeweils eine dem Kopplungsabschnitt 24 zuweisende, hier beispielhaft konvex ausgeführte, Hinterschneidung **78** auf.

Der zweite Wandschenkel 32 steht in Richtung der Gehäuselängsachse 20, d.h., in axialer Richtung, über den ersten Wandschenkel 30 hervor. Eine Länge I des zweiten Wandschenkels 32 beträgt ungefähr das 4 fache der Länge I' des ersten Wandschenkels 30. Die Länge I des zweiten Wandschenkels 32 kann grundsätzlich das zwei- bis sechsfache der Länge I' des ersten Wandschenkels 30 betragen.

Wie aus Fig. 9 hervorgeht, weist der zweite Wandschenkel 32 ungefähr auf axialer Höhe des freien Endes **80** des ersten Wandschenkels 30 eine Sollbruchstelle **82** auf. Diese ist durch eine innenseitige und eine außenseitige Kerbe **84** des zweiten Wandschenkels 32 gebildet. Die Kerben 84 erstrecken sich in Umfangsrichtung über die gesamte Breite b des zweiten Wandschenkels 32. Die Sollbruchstelle 82 kann auch in anderer Weise ausgeführt sein.

Das Innengewindesegment 34 des ersten Wandschenkels 30 erstreckt sich in axialer Richtung bis zum freien Ende 80 des ersten Wandschenkels 30. Bei dem zweiten Wandschenkel 32 erstreckt sich das Innengewindesegment 34 bis zur Sollbruchstelle 82.

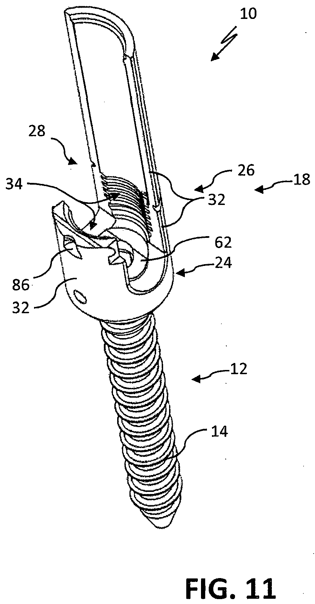

**Fig. 11** zeigt die Knochenschraube in einer perspektivischen Ansicht. Der erste Wandschenkel 30 weist außenseitig eine mit **86** bezeichnete Arretierausnehmung für ein Repositionsinstrument auf.

## Patentansprüche

1. Knochenschraube (10) für Implantate zur Korrektur und Stabilisierung der Wirbelsäule, umfassend:
- einen Gewindeschaft (12) mit einem Schraubenkopf (16), der ein Antriebsprofil (42) für ein Drehwerkzeug (120) aufweist, und
- ein Kopfgehäuse (18) mit einer Stabaufnahme (28) für einen Fixierstab (88), wobei das Kopfgehäuse (18) am Schraubenkopf (16) relativ zur Längsachse (20) des Gewindeschafts (12) der Knochenschraube (10) auslenkbar und um seine Gehäuselängsachse (20) frei drehbar am Schraubenkopf (18) angeordnet ist, wobei
das Antriebsprofil (42) von einem Einsatzteil (44) gebildet ist, das in einer Lageraufnahme (48) des Schraubenkopfs (16) angeordnet ist, wobei das Einsatzteil (44) mit dem Schraubenkopf (16) in Richtung um die Längsachse (22) des Gewindeschafts der Knochenschraube (10) drehfest verkoppelt ist und wobei
das Einsatzteil (44) am Kopfgehäuse (18) geführt gehalten und gemeinsam mit diesem gegenüber der Längsachse (20) auslenkbar ist, wobei das Kopfgehäuse (18) über einen innenspannenden geschlitzten Federring (46) an dem Schraubenkopf (16) axial lagegesichert ist, wobei das Kopfgehäuse (18) im Bereich seiner Schraubenkopfaufnahme (64) innenseitig eine Ringnut (66) aufweist, in die der Federring (46) beim axialen Einführen des Schraubenkopfs (16) in die Schraubenkopfaufnahme (64) des Kopfgehäuses (18) hinein ausweichen kann, wobei innerhalb der Ringnut (66) in deren gewindeschaftseitigen Bereich ein Vorsprung (68) für eine radiale Begrenzung einer Aufweitung des Federrings (46) angeordnet ist und wobei
die Schraubenkopfaufnahme (64) mit einem axialen Anschlag für den Federring versehen ist, der durch einen Seitenwandvorsprung (70) des
Kopfgehäuses (18) gebildet ist und durch den der Federring (46) gegenüber einem axialen Entfernen oder Herausfallen aus der Schraubenkopfaufnahme (64) des Kopfgehäuses (18) gesichert ist, **dadurch gekennzeichnet, dass**
das Einsatzteil (44) zwei Lagerzapfen (52) aufweist, die sich jeweils in radialer Richtung vom Einsatzteil (44) wegerstrecken, wobei die Lagerzapfen (52) in Seitenwandausnehmungen (58') eines in der Lageraufnahme (48) verschwenkbar angeordneten Kardanrings (72) eingreifen.

2. Knochenschraube nach Anspruch 1, **dadurch gekennzeichnet, dass** die Seitenwandausnehmungen (58') zum freien Rand (60) des Schraubenkopfs (16) hin offen sind.

3. Knochenschraube nach einem der vorhergehenden Ansprüche, **dadurch gekennzeichnet, dass** das Einsatzteil (44) zumindest abschnittsweise eine sphärische oder eine vielflächig facettierte Außenseite (50) und die Lageraufnahme (48) des Schraubenkopfs (16) eine Seitenwand (56) aufweist, die zumindest abschnittsweise gewölbt ist.

4. Knochenschraube nach einem der vorhergehenden Ansprüche, **dadurch gekennzeichnet, dass** das Einsatzteil (44) mit einem, vorzugsweise ringbundförmigen, Führungsabschnitt (62) am Kopfgehäuse (18) geführt gehalten ist.

5. Knochenschraube nach einem der vorhergehenden Ansprüche, **dadurch gekennzeichnet, dass** das Einsatzteil (44) mittels des Kopfgehäuses (18) in der Lageraufnahme (48) des Schraubenkopfs (16) axial lagegesichert ist.

6. Knochenschraube nach einem der vorhergehenden Ansprüche, **dadurch gekennzeichnet, dass** das Kopfgehäuse (18)
- einen ringförmigen Kopplungsabschnitt (24) zum Verkoppeln des Kopfgehäuses (18) mit dem Schraubenkopf (16) der Knochenschraube (10) aufweist,
- wobei die Stabaufnahme (28) des Kopfgehäuses (18) von einem ersten und einem zweiten Wandschenkel (30, 32) seitlich begrenzt ist, welche sich in Richtung einer Gehäuselängsachse (20) vom Kopplungsabschnitt (24) wegerstrecken, wobei
an den Wandschenkeln (30, 32) Innengewindesegmente (34) für eine Fixierschraube (90) angeordnet sind, wobei
der zweite Wandschenkel (32) den ersten Wandschenkel (30) in axialer Richtung überragt und wobei
der erste Wandschenkel (30) an seinem freien Randabschnitt (74) mit einem Kopplungsmittel (76) zum Ankoppeln eines Repositionsinstruments (100) ausgebildet ist.

7. Knochenschraube nach Anspruch 6, **dadurch gekennzeichnet, dass** der zweite Wandschenkel (32) eine Sollbruchstelle (82) aufweist, die axial im Wesentlichen auf Höhe des freien Endes (80) des ersten Wandschenkels (30) angeordnet ist.

8. Knochenschraube nach Anspruch 6 oder 7 , **dadurch gekennzeichnet, dass** sich das Innengewindesegment (34) des ersten Wandschenkels (30) in axialer Richtung bis zum freien Rand (80) des ersten Wandschenkels (30) und dass sich das Innengewindesegment (34) des zweiten Wandschenkels (32) bis zu der Sollbruchstelle (82) erstreckt.

## Claims

1. Bone screw (10) for implants for correcting and stabilizing the spinal column, said bone screw (10) comprising:
- a threaded shank (12) with a screw head (16), which has a driving profile (42) for a turning tool (120), and
- a head housing (18) with a rod recess (28) for a fixing rod (88), wherein the head housing (18) on the screw head (16) is deflectable relative to the longitudinal axis (20) of the threaded shank (12) of the bone screw (10) and is arranged on the screw head (18) so as to be freely rotatable about its housing longitudinal axis (20), wherein
the driving profile (42) is formed by an insert part (44) which is arranged in a bearing recess (48) of the screw head (16), wherein
the insert part (44) is coupled to the screw head (16) for conjoint rotation in a direction about the longitudinal axis (22) of the threaded shank of the bone screw (10), and wherein
the insert part (44) is held and guided on the head housing (18) and, together with the latter, is deflectable with respect to the longitudinal axis (20),
wherein the head housing (18) is secured axially in position on the screw head (16) via an inwardly tensioning, slotted spring ring (46), wherein
the head housing (18) has on the inside, in the area of its screw-head recess (64), an annular groove (66) into which the spring ring (46) can move during the axial insertion of the screw head (16) into the screw-head recess (64) of the head housing (18), wherein
a projection (68) for radially limiting an expansion of the spring ring (46) is arranged inside the annular groove (66), in the threaded shank area thereof, and wherein
the screw-head recess (64) is provided with an axial abutment for the spring ring, which axial abutment is formed by a side-wall projection (70) of the head housing (18), and by which the spring ring (46) is secured against axial removal from or falling out of the screw-head recess (64) of the head housing (18),
**characterized in that**
the insert part (44) has two bearing pins (52) which each extend in a radial direction away from the insert part (44), wherein the bearing pins (52) engage in side-wall recesses (58') of a cardan ring (72) arranged pivotably in the bearing recess (48).

2. Bone screw according to claim 1, **characterized in that** the side-wall recesses (58') are open towards the free edge (60) of the screw head (16).

3. Bone screw according to one of the preceding claims, **characterized in that** the insert part (44) has at least in parts a spherical or a multifacetted outer face (50), and the bearing recess (48) of the screw head (16) has a side wall (56) which is curved at least in parts.

4. Bone screw according to one of the preceding claims, **characterized in that** the insert part (44) is held and guided on the head housing (18) by a
guide portion (62), preferably in the shape of an annular collar.

5. Bone screw according to one of the preceding claims, **characterized in that** the insert part (44) is secured axially in position in the bearing recess (48) of the screw head (16) by means of the head housing (18).

6. Bone screw according to one of the preceding claims, **characterized in that** the head housing (18)
- has an annular coupling portion (24) for coupling the head housing (18) to the screw head (16) of the bone screw (10),
- wherein the rod recess (28) of the head housing (18) is delimited laterally by a first and a second wall leg (30, 32), which wall legs extend away from the coupling portion (24) in the direction of a housing longitudinal axis (20), wherein
internal thread segments (34) for a fixing screw (90) are arranged on the wall legs (30, 32),
wherein
the second wall leg (32) protrudes beyond the first wall leg (30) in the axial direction, and wherein
the first wall leg (30), at the free edge portion (74) thereof, is formed with a coupling means (76) for coupling a repositioning instrument (100).

7. Bone screw according to Claim 6, **characterized in that** the second wall leg (32) has a predetermined breaking point (82), which is arranged axially substantially at the height of the free end (80) of the first wall leg (30).

8. Bone screw according to Claim 6 or 7, **characterized in that** the internal thread segment (34) of the first wall leg (30) extends axially as far as the free edge (80) of the first wall leg (30), and **in that** the internal thread segment (34) of the second wall leg (32) extends as far as the predetermined breaking point (82).

## Revendications

1. Vis ostéochirurgicale (10) destinée à des implants de correction et de stabilisation de la colonne vertébrale, comprenant :
- une tige filetée (12), associée à une tête (16) de ladite vis qui est pourvue d'un profil d'entraînement (42) dévolu à un outil rotatif (120), et
- un réceptacle frontal (18) muni d'un logement (28) affecté à une broche (88) de blocage à demeure, ledit réceptacle frontal (18) pouvant être dévié, sur la tête (16) de la vis, par rapport à l'axe longitudinal (22) de la tige filetée (12) de ladite vis ostéochirurgicale (10), et étant disposé à rotation libre autour de son axe longitudinal (20) sur ladite tête (16) de la vis, sachant que
le profil d'entraînement (42) est formé par une pièce intégrée (44) située dans un logement de montage (48) de la tête (16) de la vis,
la pièce intégrée (44) étant assujettie en rotation à ladite tête (16) de la vis, avec orientation directionnelle autour de l'axe longitudinal (22) de la tige filetée de ladite vis ostéochirurgicale (10), et sachant que
ladite pièce intégrée (44) est maintenue guidée sur le réceptacle frontal (18) et peut être déviée, conjointement à ce dernier, par rapport à l'axe longitudinal (20),
le réceptacle frontal (18) étant bloqué axialement à demeure, sur la tête (16) de ladite vis, par l'intermédiaire d'une bague élastique fendue (46) à effet de serrage intérieur, sachant que
ledit réceptacle frontal (18) est intérieurement doté, dans la région de son logement (64) recevant la tête de la vis, d'une rainure annulaire (66) dans laquelle ladite bague élastique (46) peut pénétrer par déclic lors de l'introduction axiale de la tête (16) de la vis dans le logement (64) dudit réceptacle frontal (18) recevant ladite tête de la vis,
une saillie (68), ciblant une limitation d'une expansion de ladite bague élastique (46) dans le sens radial, étant ménagée à l'intérieur de ladite rainure annulaire (66), dans la région de cette dernière située côté tige filetée, et sachant que
ledit logement (64), recevant la tête de la vis, est muni d'une butée axiale qui est dédiée à la bague élastique, est formée par une protubérance (70) de la paroi latérale du réceptacle frontal (18), et par l'intermédiaire de laquelle ladite bague élastique (46) est arrêtée à l'encontre d'une dissociation axiale ou d'une chute hors dudit logement (64) dudit réceptacle frontal (18) qui reçoit ladite tête de la vis,
**caractérisée par le fait que**
la pièce intégrée (44) comporte deux tourillons (52) s'étendant respectivement à partir de ladite pièce intégrée (44), dans le sens radial, lesdits tourillons (52) pénétrant dans des évidements (58') de la paroi latérale d'un anneau de Cardan (72) disposé, de manière pivotante, dans le logement de montage (48).

2. Vis ostéochirurgicale selon la revendication 1, **caractérisée par le fait que** les évidements (58') de la paroi latérale sont ouverts en direction du bord libre (60) de la tête (16) de ladite vis.

3. Vis ostéochirurgicale selon l'une des revendications précédentes, **caractérisée par le fait que** la pièce intégrée (44) présente, au moins par zones, une face extérieure (50) sphérique ou polyédrique, et le logement de montage (48) de la tête (16) de ladite vis est muni d'une paroi latérale (56) bombée, au moins par zones.

4. Vis ostéochirurgicale selon l'une des revendications précédentes, **caractérisée par le fait que** la pièce intégrée (44) est maintenue guidée sur le réceptacle frontal (18) par un tronçon de guidage (62) revêtant, de préférence, la forme d'une collette annulaire.

5. Vis ostéochirurgicale selon l'une des revendications précédentes, **caractérisée par le fait que** la pièce intégrée (44) est bloquée axialement à demeure, au moyen du réceptacle frontal (18), dans le logement de montage (48) de la tête (16) de ladite vis.

6. Vis ostéochirurgicale selon l'une des revendications précédentes, **caractérisée par le fait que** le réceptacle frontal (18) comporte
- un tronçon annulaire d'accouplement (24), en vue de l'accouplement dudit réceptacle frontal (18) avec la tête (16) de ladite vis ostéochirurgicale (10),
- le logement (28) dudit réceptacle frontal (18), affecté à une broche, étant délimité latéralement par des première et seconde ailes de cloisonnement (30, 32) qui partent dudit tronçon d'accouplement (24) en direction d'un axe longitudinal (20) dudit réceptacle, sachant que
des segments (34) à filetages intérieurs, affectés à une vis (90) de blocage à demeure, sont ménagés sur lesdites ailes de cloisonnement (30, 32),
la seconde aile de cloisonnement (32) dépassant au-delà de la première aile de cloisonnement (30), dans le sens axial, et sachant que
ladite première aile de cloisonnement (30) est pourvue, dans sa région marginale libre (74), d'un moyen d'accouplement (76) dévolu à l'accouplement d'un instrument de repositionnement (100).

7. Vis ostéochirurgicale selon la revendication 6, **caractérisée par le fait que** la seconde aile de cloisonnement (32) présente une zone (82) de rupture par destination placée pour l'essentiel, dans le sens axial, à la hauteur de l'extrémité libre (80) de la première aile de cloisonnement (30).

8. Vis ostéochirurgicale selon la revendication 6 ou 7, **caractérisée par le fait que** le segment (34) à filetage intérieur de la première aile de cloisonnement (30) s'étend, dans le sens axial, jusqu'au bord libre (80) de ladite première aile de cloisonnement (30) ; et **par le fait que** le segment (34) à filetage intérieur de la seconde aile de cloisonnement (32) s'étend jusqu'à la zone (82) de rupture par destination.
